# FASCICULE DE BREVET EUROPEEN

(11) **EP 3 766 581 B1**
(45) Date de publication et mention de la délivrance du brevet: **22.12.2021**
(21) Numéro de dépôt: 20184842.1
(22) Date de dépôt: 09.07.2020
(51) Int. Cl.: B01L 3/00, B01L 7/00, C12Q 1/6844

(54) **SYSTÈME MICRO-FLUIDIQUE INCLUANT UNE CHAMBRE DE RÉACTION D'AMPLIFICATION**
MIKROFLUIDISCHES SYSTEM MIT VERSTÄRKUNGSREAKTIONSKAMMER
MICRO-FLUIDIC SYSTEM INCLUDING AN AMPLIFICATION REACTION CHAMBER

(30) Priorité: 17.07.2019 FR 1908033
(43) Date de publication de la demande: 20.01.2021
(73) Titulaire: Commissariat à l'Energie Atomique et aux Energies Alternatives, 75015 Paris (FR)
(72) Inventeur: BORDY, Thomas, 38054 GRENOBLE Cedex 09 (FR); BOURDAT, Anne-Gaëlle, 38054 GRENOBLE Cedex 09 (FR); TOUTAIN, Rémi, 38054 GRENOBLE Cedex 09 (FR)
(74) Mandataire: INNOV-GROUP

(56) Documents cités:
- EP-A1- 3 173 469
- EP-A1- 3 222 989
- JP-A- 2009 098 039
- US-A1- 2010 009 431
- US-A1- 2017 113 221

## Description

### Domaine technique de l'invention

La présente invention se rapporte à un système micro-fluidique, plus particulièrement à l'architecture d'une chambre située dans un dispositif du système et destinée à la mise en œuvre d'une réaction d'amplification.

### Etat de la technique

La réaction de type qPCR consiste en une amplification d'une séquence d'ADN ou d'ARN ciblée (représentative d'un organisme en particulier) couplée à un intercalant ou à une sonde produisant une fluorescence détectable par un appareil optique en cas d'amplification de cette séquence. Ainsi, si le niveau de fluorescence augmente durant la réaction, cela signifie que la réaction d'amplification se produit et que donc l'ADN ou l'ARN de l'organisme cible était bien présent. Néanmoins, en cas d'absence de réaction, il faut être capable d'affirmer que cela est dû à l'absence de l'organisme recherché et non à une inhibition de la réaction d'amplification, ce qui donnerait lieu à un faux négatif. Les enzymes responsables de la réaction d'amplification sont en effet sensibles à de nombreux inhibiteurs apportés par l'échantillon analysé.

Afin de garantir que l'absence d'amplification signifie bien l'absence de la cible, des contrôles internes de réactions sont mis en place. La plupart du temps, il s'agit d'une autre cible d'ADN ajoutée volontairement au test qui va être amplifiée simultanément avec l'échantillon d'intérêt. Il faut alors être capable de discriminer les deux réactions. Plusieurs stratégies sont utilisées dans l'industrie :
- L'une d'elles pourrait être d'utiliser une partie de l'échantillon pour réaliser le contrôle en parallèle, en tant que réaction indépendante. Ceci oblige à fractionner l'échantillon initial entrainant une perte de la sensibilité/représentativité du test. L'avantage est qu'il est possible d'utiliser n'importe quelle technique de détection d'amplification pour ce contrôle (exemple : intercalant ADN fluorescent).
- Afin de ne pas diviser l'échantillon, une autre stratégie consiste à réaliser le contrôle dans la même réaction que la séquence ciblée. Cette solution permet de tester l'intégralité de l'échantillon mais n'est pas compatible avec toutes les techniques de détection, notamment avec l'utilisation d'agent intercalent non spécifique, d'une séquence ou de tout autre mode de détection non spécifique de séquence (colorimetrie, pH metrie...).

La demande de brevet EP0586112A2 et le brevet US6312930B1 décrivent chacun une méthode de détection permettant d'éliminer les faux négatifs, par ajout d'une cible de contrôle. Le document EP 3 173 469 A1 concerne un système de détection PCR multiplexe. Le document JP 2009 098039 A concerne un dispositif d'analyse pour lecture optique. Le document US 2010/009431 A1 concerne un dispositif microfluidique.

L'invention vise donc à proposer un système micro-fluidique doté d'une solution intégrée pour contrôler la réaction d'amplification ou pour identifier plusieurs cibles lors d'une même analyse. L'invention concerne également un procédé d'analyse mettant en œ uvre ledit système.

### Exposé de l'invention

Ce but est atteint par un système micro-fluidique destiné à l'analyse d'un échantillon biologique contenant des espèces biologiques, ledit système comprenant :
- Un dispositif de détection optique comprenant une source configurée pour émettre un signal optique et au moins un capteur présentant une surface de capture définissant une zone de lecture du signal optique transparente,
- Un dispositif micro-fluidique qui comporte :
   ∘ Un support dans lequel est réalisé une chambre dite d'amplification dans laquelle peut être réalisée une réaction d'amplification,
   ∘ Un canal d'entrée débouchant dans ladite chambre d'amplification,
- La chambre d'amplification comportant au moins une première zone située dans la zone de lecture du capteur et au moins une proéminence formant une alcôve dans laquelle est placée un composé de contrôle interne de la réaction d'amplification, la dite alcôve étant agencée pour être située en dehors de la zone de lecture du capteur ou configurée pour être opaque audit signal optique.

Selon une particularité, la chambre d'amplification comporte un premier volume présentant une première section et un deuxième volume présentant une deuxième section rétrécie par rapport à ladite première section de manière à former une proéminence, ladite proéminence formant ladite alcôve.

Selon une autre particularité, ledit support comporte plusieurs strates superposées et ladite chambre d'amplification est réalisée par au moins deux desdites strates superposées, dites strate supérieure et strate inférieure, ladite alcôve est réalisée dans l'une desdites deux strates uniquement.

Selon une autre particularité, ladite proéminence formant ladite alcôve est réalisée dans la strate inférieure.

Selon une autre particularité, la chambre d'amplification comporte une cavité principale réalisée dans la strate supérieure et une ou plusieurs cavités secondaires réalisées dans la strate inférieure et formant chacune une autre alcôve de ladite chambre.

Selon l'invention, le composé de contrôle interne de réaction comporte une séquence ADN connue ou un ensemble d'amorces ADN visant une cible ADN prédéfinie, permettant son amplification selon la méthode d'amplification utilisée.

Selon une autre particularité, la deuxième zone présente au moins une partie opaque configurée pour ne pas laisser passer ledit signal optique.

L'invention concerne également un procédé d'analyse d'un échantillon biologique contenant des espèces biologiques, ledit procédé étant mis en œuvre par un système tel que défini ci-dessus et consistant à :
- Injecter un échantillon fluidique dans la chambre d'amplification du dispositif micro-fluidique,
- Détecter grâce au capteur la présence d'un composé cible contenu dans ledit échantillon fluidique et situé dans la première zone de la chambre d'amplification,
- Détecter, avec un décalage temporel, la présence du composé de contrôle interne de réaction dans la première zone de la chambre d'amplification.

### Brève description des figures

D'autres caractéristiques et avantages vont apparaître dans la description détaillée qui suit faite en regard des dessins annexés dans lesquels :
- La figure 1 représente un diagramme montrant toutes les étapes d'un procédé de préparation et d'analyse d'un échantillon biologique par amplification biomoléculaire.
- La figure 2 représente un dispositif micro-fluidique à deux unités, permettant de mettre en œuvre les étapes du procédé de préparation et d'analyse défini en figure 1.
- La figure 3 représente un exemple de réalisation du dispositif de la figure 2, vu en éclaté.
- Les figures 4A et 4B illustrent un exemple de réalisation de la chambre d'amplification pouvant être employée dans le dispositif de la figure 2.
- Les figures 5A à 5C représentent différentes variantes de réalisation de la chambre d'amplification.

### Description détaillée d'au moins un mode de réalisation

Le dispositif micro-fluidique de l'invention est destiné à l'analyse d'un échantillon biologique. Cet échantillon biologique se présente par exemple sous la forme d'un fluide qui contient des espèces biologiques contenant un matériel biologique à étudier.

Par espèces biologiques, on entend notamment des micro-organismes, des cellules, des spores, des virus... Par matériel biologique à étudier, on entend par exemple des molécules d'acide nucléique (ARN, ADN) issues d'une cellule, des protéines, des lipopolysaccharides (LPS), des acides lipotéichoïques (LTA)...

Par fluide, on entend notamment un liquide, un gaz... Le liquide pourra présenter différents degrés de viscosité et pourra par exemple se présenter sous la forme d'une pâte ou d'un gel.

Dans la suite de la description, les termes "inférieur", "supérieur", "haut" et "bas" employés sont à comprendre en prenant comme référence un axe principal (X) qui est vertical.

Dans la suite de la description, les termes "externe", "extérieur", "interne", "intérieur", doivent être compris en prenant comme référence les chambres du dispositif qui seront décrites ci-dessous.

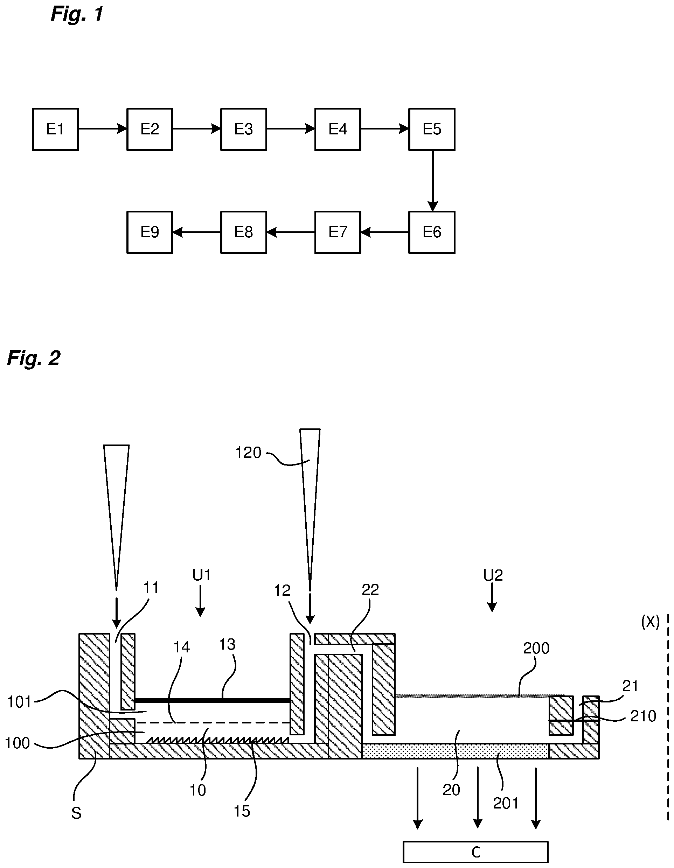

En référence à la figure 1, l'analyse complète d'un échantillon biologique tel que défini ci-dessus peut comporter les étapes suivantes réalisées de manière successive :
- E1 : Concentration des espèces biologiques présentes dans l'échantillon biologique,
- E2 : Lavage pour purification, pour élimination des interférents de culture,
- E3 : Apport d'un milieu de culture,
- E4 : Culture des espèces biologiques,
- E5 : Suivi optique de croissance lors de la culture et comptage des colonies,
- E6 : Lavage, pour élimination des inhibiteurs de PCR,
- E7 : Lyse mécanique des espèces biologiques présentes dans l'échantillon en vue d'en extraire un matériel biologique à étudier,
- E8 : Séparation entre le matériel biologique à étudier et les polluants présents,
- E9 : Une détection de présence de pathogènes dans le matériel biologique par amplification biomoléculaire de type qPCR, LAMP, RPA et détection optique tel que par exemple fluorescence, colorimétrie, imagerie holographique, turbidimétrie, pHmétrie en liaison avec la réaction d'amplification.

Bien entendu, toutes ces étapes ne sont pas forcément toutes réalisées dans le dispositif, le procédé pouvant être limité à certaines étapes seulement.

Lors de l'étape de concentration, l'échantillon biologique, par exemple sous forme liquide, comprenant les espèces biologiques, est injecté dans une chambre pour passer à travers un filtre. La partie liquide de l'échantillon et toutes les particules/molécules qui passent à travers le filtre sont récupérées par un canal d'évacuation et écartées de l'analyse. Les espèces biologiques sont alors concentrées dans un espace de la chambre.

Une solution de lavage/rinçage peut être injectée pour laver les espèces biologiques présentes dans la chambre.

Un milieu de culture est injecté pour permettre la culture des espèces biologiques.

L'étape de suivi de croissance permet, par lecture optique, à l'aide d'un capteur C, de suivre la croissance cellulaire lors de l'étape de culture.

La lyse mécanique des espèces biologiques est mise en œuvre pour broyer les espèces biologiques présentes dans l'échantillon contre une surface d'appui rugueuse. Une fois la lyse mécanique effectuée, on dispose d'un matériel biologique à étudier, formé par exemple de molécules d'ADN et de polluants.

La séparation entre le matériel biologique à étudier et les polluants est réalisée en injectant une solution liquide contenant des réactifs d'amplification, pour éluer le matériel biologique à étudier. Une partie de la solution liquide injectée emporte ainsi le matériel biologique à étudier, par exemple les molécules d'ADN, qui passe à travers le filtre.

Une fois que la séparation entre les polluants et le matériel biologique à étudier est effectuée, la réaction d'amplification du matériel biologique permet de détecter la présence d'un pathogène dans le matériel biologique qui a été séparé. La réaction d'amplification est mise en œuvre en ajoutant un mélange d'amplification et en chauffant une chambre dans laquelle est placée l'échantillon. La température à laquelle est chauffée la chambre dépend du type de réaction d'amplification mise en oeuvre. Il pourra s'agir de tous types de réaction d'amplification, par exemple LAMP ("Loop-Mediated Isothermal Amplification"), PCR ("Polymerase Chain Reaction"), NASBA ("Nucleic Acid Sequence Based Amplification"), RPA ("Recombinase Polymerase Amplification") ... Pour une amplification de type LAMP, le chauffage est réalisé à une température avantageusement comprise entre 60°C et 65°C. Cette réaction permet d'amplifier les molécules du matériel biologique à détecter, par exemple les molécules d'ADN. Lors de la réaction d'amplification du matériel biologique, il s'agit de détecter si un pathogène est présent. Pour cela, il est possible d'employer différentes méthodes, comme par exemple la colorimétrie, la fluorescence, l'électrochimie, la pHmétrie, la mesure de turbidité. Toute autre méthode de détection pourrait être envisagée. Pour une méthode de détection de type PHmétrie, des électrodes de détection de pH pourraient être intégrées au dispositif.

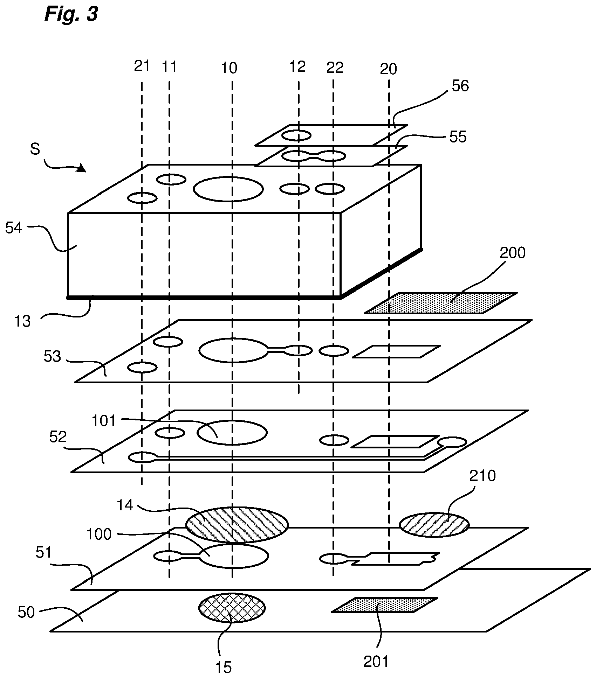

Un dispositif micro-fluidique permettant de réaliser les étapes décrites ci-dessus est représenté sur la figure 2. Ce dispositif comporte un unique support S rigide. La figure 3 décrite ci-dessous donne un exemple de réalisation du dispositif.

Ce support S rigide intègre un réseau micro-fluidique adapté à la mise en œuvre des étapes du procédé d'analyse. On verra que le réseau micro-fluidique peut prendre différentes architectures selon la configuration du procédé d'analyse qui est mise en œuvre.

Le support S comporte avantageusement une paroi inférieure plane et une architecture à plusieurs couches superposées suivant ledit axe principal, empilées sur sa paroi inférieure.

Le réseau micro-fluidique du dispositif comporte au moins deux unités U1, U2

La première unité U1 comporte une première chambre 10 ménagée dans ledit support. Cette chambre 10 présente un volume non nul et est délimitée par des parois du support S.

La première unité U1 comporte un premier canal 11 ménagé dans le support pour injecter des fluides dans la première chambre 10 ou pour évacuer des fluides en dehors de cette première chambre. Le premier canal 11 comporte une première extrémité comportant une ouverture ménagée par exemple à travers une paroi supérieure du support S et une deuxième extrémité qui débouche dans ladite première chambre 10. La première extrémité du premier canal est par exemple agencée verticalement et sa deuxième extrémité débouche par exemple horizontalement dans la première chambre 10.

La première unité U1 comporte un deuxième canal 12. Ce deuxième canal 12 comporte également une première extrémité qui communique avec l'extérieur, formant une ouverture réalisée par exemple à travers une paroi supérieure du support S et une deuxième extrémité qui communique avec l'espace formé par la première chambre 10. Par ce deuxième canal 12, on peut également injecter des fluides dans ladite première chambre ou évacuer des fluides en dehors de ladite première chambre. Sa première extrémité est par exemple agencée verticalement et sa deuxième extrémité horizontalement. La première chambre 10 est placée entre le premier canal 11 et le deuxième canal 12.

Vers le haut, la première chambre 10 peut être fermée par une membrane 13 souple et étirable. Au niveau de la première chambre, une paroi supérieure du support comporte ainsi une ouverture qui est recouverte de manière hermétique par ladite membrane 13. La membrane 13 est ainsi ancrée dans le support par toute solution de fixation adaptée, par exemple par collage. Cette membrane 13 sera par exemple composée d'un film, par exemple de type MicroAmp, 3M (marques déposées), d'épaisseur, de dimensions et de constitution adaptées pour se déformer de manière hyper-élastique, par rapport à ses points d'ancrage, au moins jusqu'au fond de la première chambre.

La membrane 13 est apte à se déformer de manière réversible entre plusieurs configurations. Elle peut s'étirer par déformation hyper-élastique vers l'extérieur du support S, se rétracter à l'intérieur de la première chambre 10 par compression, ou être au repos. Par matériau hyper-élastique, on entend un matériau capable de présenter une surface apte à passer d'une première superficie à une deuxième superficie, la deuxième superficie étant égale à au moins 5 fois la première superficie, par exemple à 10 fois ou même à 50 fois la première superficie.

La première unité U1 comporte également un filtre 14 transversal agencé dans ladite première chambre 10 et séparant ladite première chambre 10 en deux espaces 100, 101. Les deux espaces sont par exemple superposés et désignés ainsi espace inférieur 100 situé sous le filtre 14 et espace supérieur 101 situé au-dessus du filtre 14. Ce filtre 14 est préférentiellement réalisé en tout ou partie sous la forme d'un film souple et fin, tiré dans l'espace formé par la chambre de manière à ne permettre le passage d'un espace à l'autre que par les pores du filtre 14. Le film présente une déformabilité élastique lui permettant de s'étirer lors de l'exercice d'une force d'appui dans une direction sensiblement verticale, cette déformabilité élastique ayant un niveau suffisant pour atteindre le fond de la chambre 10. Le filtre 14 présente un diamètre moyen de pores compris entre 0.2 µm et 50 µm, par exemple compris entre 0.2 µm et 1 µm pour la séparation de microorganismes. Le diamètre des pores est bien entendu adapté pour assurer une séparation entre différentes espèces biologiques présentes dans l'échantillon. Le filtre 14 sera par exemple composé d'un film d'épaisseur, de dimensions et de constitution adaptée pour se déformer jusqu'au fond de la chambre 10 par rapport à ses points d'ancrage. Il pourra comporter les mêmes caractéristiques d'hyper-élasticité que la membrane.

Selon une particularité, le premier canal 11 débouche dans l'espace supérieur 101 de la première chambre 10 et le deuxième canal 12 débouche dans l'espace inférieur 100 de la première chambre 10. Les embouchures des deux canaux sont donc séparées par le filtre 14 agencé dans la chambre.

En référence à la figure 2, la première unité U1 peut avantageusement comporter une surface d'appui rugueuse 15 agencée sur le fond de la première chambre 10. Cette surface d'appui rugueuse 15 s'étend sur une partie majoritaire du fond de la première chambre. Elle comporte un paramètre de rugosité de surface moyen compris entre 0.1µm et 10 µm, préférentiellement compris entre 0.2 µm et 3 µm. Cette surface d'appui rugueuse 15 est destinée à permettre une lyse mécanique des espèces biologiques présentes dans un échantillon biologique placé dans le dispositif. Préférentiellement, la lyse mécanique est réalisée en broyant lesdites espèces biologiques, par abrasion sur ladite surface d'appui rugueuse. L'opération de broyage est mise en œuvre par un mouvement de friction des espèces biologiques contre la surface d'appui rugueuse, en employant un organe de broyage adapté. Cet organe sera par exemple une spatule ou une tige, par exemple en matériau plastique ou métallique. Cet organe est appliqué de l'extérieur de la chambre 10 et son extrémité est appliquée contre la surface externe de la membrane 13 de manière à étirer la membrane 13 et le filtre 14 vers le fond de la première chambre 10 et ainsi frictionner les espèces biologiques présentes dans un échantillon contre la surface d'appui rugueuse 15.

La deuxième unité U2 du dispositif comporte pour sa part une deuxième chambre 20 de volume non nul, délimitée par des parois du support S. La deuxième unité U2 comporte également un troisième canal 21 ménagé dans ledit support. Ce troisième canal 21 comporte une première extrémité comportant une ouverture ménagée par exemple à travers une paroi supérieure du support et une deuxième extrémité qui débouche uniquement dans ladite deuxième chambre 20. La première extrémité de ce troisième canal 21 est par exemple agencée verticalement et sa deuxième extrémité débouche par exemple horizontalement dans la deuxième chambre 20. La première extrémité de ce troisième canal est par exemple obturée par une membrane 210 hydrophobe, c'est-à-dire imperméable au liquide mais perméable au gaz tel que l'air. Cette membrane 210 hydrophobe peut être réalisée dans un matériau de type PTFE (Polytétrafluoroéthylène).

Deux parois transversales du support, avantageusement une paroi supérieure 200 et une paroi inférieure 201 parallèles, délimitant partiellement la deuxième chambre 20, sont réalisées dans un matériau transparent, permettant ainsi de réaliser une lecture optique à travers le volume de la deuxième chambre. Par le terme "transparent", on entend que le matériau employé est au moins partiellement transparent à la lumière visible, de manière à laisser passer au moins 80% de cette lumière. Il faut ainsi comprendre qu'il sera suffisamment transparent pour voir l'intérieur de la chambre. La paroi inférieure peut être réalisée en verre et la paroi supérieure peut être formée d'un adhésif amovible collé pour fermer ladite deuxième chambre du côté supérieur.

Selon une particularité de l'invention, le dispositif comporte également un premier canal de transfert 22 ménagé dans ledit support. Ce premier canal de transfert 22 est destiné à relier la première chambre 10, plus précisément son espace inférieur 100, à la deuxième chambre 20.

De manière avantageuse, le premier canal de transfert 22 comporte une première extrémité débouchant directement dans le deuxième canal 12, formant ainsi un nœud de dérivation sur ce deuxième canal 12. Il comporte une deuxième extrémité débouchant directement dans la deuxième chambre.

Le dispositif comporte en outre des moyens d'aiguillage pouvant être par exemple agencés sur le deuxième canal 12 pour sélectionner la liaison de la première chambre :
- Vers l'extérieur uniquement via le deuxième canal uniquement ou,
- Vers la deuxième chambre uniquement à travers le premier canal de transfert.

Ces moyens d'aiguillage peuvent consister en un cône 120 amovible creux se présentant sous la forme d'un entonnoir. Lorsqu'il est inséré par sa pointe dans le deuxième canal 12, il permet la communication entre l'extérieur et la première chambre et sa paroi obture l'entrée du premier canal de transfert 22, réalisée au niveau du noeud de dérivation. Lorsqu'il est retiré, la première extrémité du deuxième canal 12 peut être obturée, par exemple à l'aide d'un adhésif 121 appliqué sur une surface du support et la liaison entre le premier canal de transfert 22 et le deuxième canal 12 est alors ouverte, permettant à un fluide de circuler entre la première chambre 10 et la deuxième chambre 20.

Bien entendu, les moyens d'aiguillage peuvent être réalisés selon d'autres variantes de réalisation. Le principe général étant de pouvoir accéder à la première chambre en obturant le canal de transfert ou permettre une liaison entre la première chambre et la deuxième chambre. Il peut ainsi s'agir d'un simple clapet qui :
- Dans une première position, permet d'autoriser l'accès au-deuxième canal en obturant l'embouchure du canal de transfert 22 au niveau du noeud de dérivation,
- Dans une deuxième position, permet d'ouvrir la liaison entre le deuxième canal 12 et le canal de transfert 22.

De manière non limitative, le dispositif peut être réalisé selon l'architecture représentée sur la figure 3.

Sur cette figure 3, le support S comporte les particularités suivantes :
- Le support comporte une lame 50, par exemple en verre ou en en matériau plastique, de type PMMA ou COC ;
- La lame 50 est recouverte dans une première zone d'une surface abrasive de manière à former sur une partie de sa face supérieure la surface d'appui rugueuse 15 dédiée à la lyse ;
- Dans au moins une deuxième zone la lame 50 est transparente pour former la paroi inférieure 201 transparente de la deuxième chambre 20, destinée à la lecture optique ;
- Une première couche 51 portant une première empreinte micro-fluidique est déposée sur la face supérieure de la lame 50, cette première empreinte comportant une première cavité définissant l'espace inférieur 100 de la première chambre 10, une deuxième cavité définissant une partie inférieure de la deuxième chambre 20 et la partie inférieure du troisième canal 21 ; la première cavité a ses bords agencés autour de la première zone abrasive et la deuxième cavité a ses bords agencés autour de la deuxième zone de lecture ;
- Le filtre 14 est apposé sur la première couche pour recouvrir l'espace inférieur 100 de la première chambre et la membrane hydrophobe 210 est apposée sur la partie inférieure du troisième canal 21 ;
- Une deuxième couche 52 portant une deuxième empreinte micro-fluidique est déposée sur la face supérieure de la première couche 51, en recouvrant également le filtre 14 et la membrane hydrophobe 210. Cette deuxième empreinte micro-fluidique comporte une cavité formant l'espace supérieur 101 de la première chambre 10, une deuxième cavité formant une partie médiane de la deuxième chambre 20 et la partie supérieure du troisième canal 21 ;
- Une troisième couche 53 portant une troisième empreinte micro-fluidique est déposée sur la face supérieure de la deuxième couche 52 ; Cette troisième empreinte micro-fluidique comporte la partie supérieure de la première chambre 10 et la partie supérieure de la deuxième chambre 20 ;
- Une lame de verre ou en matériau plastique est dimensionnée pour recouvrir la face supérieure de la troisième couche 53 au niveau de la deuxième chambre, formant la paroi supérieure 200 transparente du support ;
- Un capot 54, par exemple en PMMA, est positionné au-dessus de la première chambre 10 ; Sur sa face inférieure, le capot comporte la membrane 13 destinée à refermer la première chambre par le dessus ;
- Le capot comporte une ouverture axiale supérieure, permettant d'accéder à la membrane 13, pour effectuer la lyse ;
- Le capot 54 comporte deux entrées/sorties fluidiques sur sa face supérieure. La première entrée/sortie est reliée à un premier canal axial traversant formé à travers la membrane 13 et les trois couches et débouchant dans la première chambre 10 pour former le premier canal 11 du support ; La deuxième entrée/sortie est reliée à un deuxième canal axial traversant formé à travers la membrane 13 et la deuxième et troisième couches et débouchant au-dessus de la membrane hydrophobe 210, pour former le troisième canal 21 du support ;
- Le support comporte enfin deux autres canaux traversants formant le premier canal de transfert 22 reliant les deux chambres 10, 20 ;
- Deux autres couches 55, 56 permettent de réaliser l'entrée du deuxième canal 12 et la jonction du canal de transfert 22 sur ce deuxième canal 12 ;

L'invention a plus particulièrement trait à la deuxième chambre 20, dite chambre d'amplification. Cette chambre d'amplification présente une architecture adaptée à la mise en œuvre de l'étape de détection décrite ci-dessus.

La chambre d'amplification présente en effet un volume interne façonné de manière adaptée, soit pour assurer un contrôle fiable de la réaction d'amplification, soit pour identifier plusieurs cibles simultanément. Dans ce dernier cas, la chambre peut être façonnée pour permettre d'identifier plusieurs cibles simultanément. Ces tests, dits multiplex, sont par exemple utilisés pour détecter des groupes d'organismes pathogènes correspondant à des symptômes cliniques proches ou à détecter une bactérie mais aussi ses potentiels gènes de résistance aux antibiotiques.

Le principe est de créer au moins une alcôve Ax (x allant de 1 à N, N correspondant au nombre d'alcôves et étant supérieur ou égal à 1) dans la chambre d'amplification, pour y loger un composé de contrôle interne de réaction, à savoir une séquence d'ADN choisie ou des amorces d'amplification visant un ADN prédéfini, adapté à la technologie d'amplification mise en œuvre (PCR, LAMP RPA...). Selon ce principe, soit l'ADN est séché dans l'alcôve, les amorces sont alors amenées par le liquide introduit par la chambre, soit les amorces sont séchées dans l'alcôve et l'ADN est amené par le liquide introduit dans la chambre.

Cette alcôve Ax est avantageusement créée en dehors de la section de la chambre dédiée à la lecture optique. La section de la chambre dédiée à la lecture optique correspond à une zone Z de lecture optique. La zone Z de lecture optique est la seule zone de la chambre visible par un capteur C. La chambre peut comporter des zones situées en dehors de cette zone de lecture optique Z, et donc en dehors du champ de lecture du capteur C.

Dans le cas d'un contrôle de réaction, le composé de contrôle interne peut être déposé dans l'alcôve Ax à une concentration connue puis séché directement dans la chambre. Il reste ainsi à demeure dans le dispositif et est prêt à l'emploi.

L'intérêt de déposer le composé non pas directement dans la zone Z de lecture optique mais en dehors de celle-ci permet, en plus d'optimiser l'espace d'échange gazeux, de différencier l'amplification de contrôle de l'amplification cible. L'amplification de contrôle sera en effet à la fois décalée temporellement et spatialement.

L'architecture du dispositif en plusieurs couches permet de réaliser la chambre d'amplification avec des strates de designs différents. La strate inférieure peut en effet comporter une ou plusieurs alcôves et les strates supérieures de la chambre définissent la section transversale totale de lecture optique de la chambre.

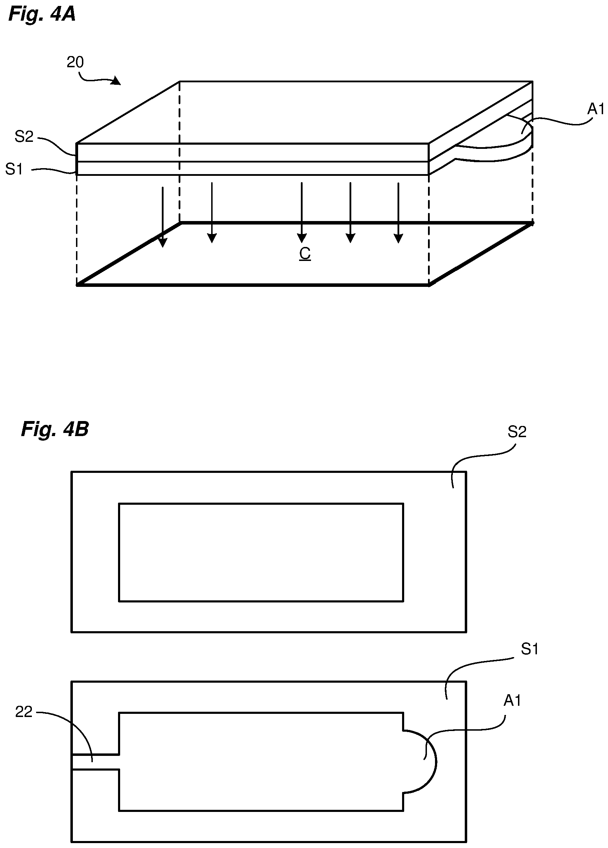

Ce principe en plusieurs strates est illustré par les figures 4A et 4B. Sur ces figures, on peut voir une strate inférieure S1 portant une alcôve A1 et une strate supérieure S2 définissant la section transversale de la zone Z de lecture optique (représentée ici de manière non limitative avec une forme rectangulaire). L'alcôve A1 est ici en forme de demi-lune. La figure 4A représente la chambre d'amplification sous la forme d'un solide et permet d'illustrer la position du capteur C par rapport à la chambre, la surface du capteur C définissant la zone Z de lecture optique de la chambre.

Comme illustré la figure 4A, l'alcôve A1 peut être située en dehors de la zone Z de lecture optique du capteur C. Sur la figure 4B, la chambre d'amplification est réalisée par superposition des deux strates S1, S2. Sur cette figure, on peut aussi voir le canal d'entrée dans la chambre, réalisé dans la strate inférieure S1 et correspondant au premier canal de transfert 22.

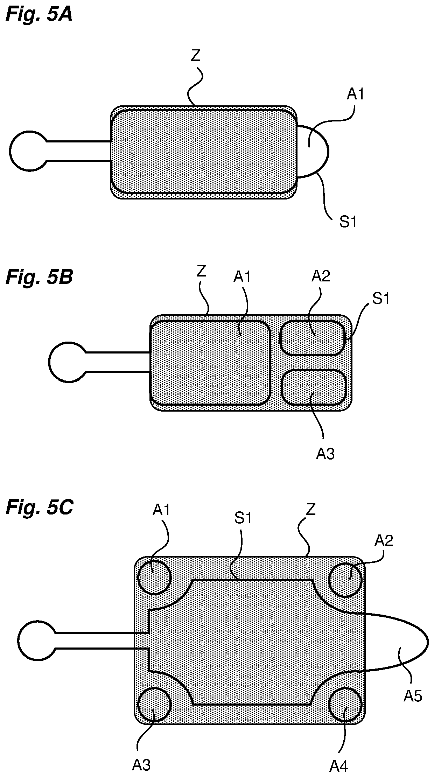

Les figures 5A à 5C montrent différentes architectures de la strate inférieure de la chambre d'amplification. Sur ces figures, la zone Z de lecture optique est représentée en grisé.

Sur la figure 5A, la strate inférieure S1 comporte une proéminence formant l'alcôve A1 allant au-delà de la section de la zone Z de lecture optique de la chambre dans le plan transversal, à l'opposé du point d'entrée de liquide dans la chambre (correspondant au design des figures 4A et 4B). Cette zone déportée permet de décaler la réaction d'amplification temporellement et spatialement.

Sur la figure 5B, la strate inférieure S1 comporte trois alcôves A1, A2, A3 distinctes formées par trois cavités permettant de loger trois amorces distinctes. Ces trois zones sont situées dans la zone Z de lecture optique et permettent de mettre en œuvre le principe de multiplexage.

La figure 5C montre une state inférieure, définissant cinq zones distinctes, quatre alcôves A1-A4 réalisées chacune sous la forme d'une cavité et une zone principale comprenant une proéminence présentant une cinquième alcôve A5. La strate supérieure de la chambre qui est située juste au-dessus définit la zone de lecture optique. La zone Z de lecture optique présente un design recouvrant les quatre alcôves A1 -A4 pour que son volume soit en communication fluidique avec celles-ci. La cinquième alcôve peut être en dehors de la zone Z de lecture optique, comme dans le design de la figure 5A.

Comme décrit ci-dessus chaque amorce peut être placée dans une alcôve distincte de la chambre, sous une forme sèche. Pour faciliter le séchage il est conseillé de reprendre les amorces dans un tampon acide (environ pH 6) et faciliter leur liaison avec le verre de la lame. Il est aussi possible d'utiliser des sucres (exemple tréhalose) pour limiter la diffusion de la goutte et potentiellement augmenter la stabilité de la séquence d'ADN séchée.

Par ailleurs, il faut noter que quelle que soit son architecture, la chambre 20 présente avantageusement des angles et contours arrondis, permettant d'optimiser la propagation du liquide dans la chambre et d'éviter la formation de bulles.

On comprend de ce qui précède que le dispositif de l'invention présente de nombreux avantages. Sa chambre d'amplification du dispositif peut permettre en effet de répondre à plusieurs objectifs :
- Optimiser la fluidique (absence de bulles) ;
- Pousser l'air hors du composant mais retenir le liquide ;
- Ne pas contaminer l'environnement ;
- Permettre le multiplexage ;
- Accueillir un contrôle interne de réaction ;
- Limiter les volumes morts ;
- Ne pas diviser l'échantillon ;

## Revendications

1. Système micro-fluidique destiné à l'analyse d'un échantillon biologique contenant des espèces biologiques, ledit système comprenant :
- Un dispositif de détection optique comprenant une source configurée pour émettre un signal optique et au moins un capteur présentant une surface de capture définissant une zone de lecture du signal optique transparente,
- Un dispositif micro-fluidique qui comporte :
∘ Un support dans lequel est réalisé une chambre dite d'amplification (20) dans laquelle peut être réalisée une réaction d'amplification,
∘ Un canal d'entrée débouchant dans ladite chambre d'amplification,
- La chambre d'amplification comportant au moins une première zone située dans la zone de lecture du capteur et au moins une proéminence formant une alcôve (Ax) dans laquelle est placée un composé qui comporte une séquence ADN connue ou un ensemble d'amorces ADN visant une cible ADN prédéfinie, permettant son amplification selon la méthode d'amplification utilisée,
**caractérisé en ce que** le composé est un composé de contrôle interne de la réaction d'amplification et **en ce que** ladite alcôve (Ax) est agencée pour être située en dehors de la zone de lecture du capteur ou configurée pour être opaque audit signal optique.

2. Système selon la revendication 1, **caractérisé en ce que** la chambre d'amplification comporte un premier volume présentant une première section et un deuxième volume présentant une deuxième section rétrécie par rapport à ladite première section de manière à former une proéminence, ladite proéminence formant ladite alcôve (Ax).

3. Système selon la revendication 1 ou 2, **caractérisé en ce que** ledit support comporte plusieurs strates (S1, S2) superposées et **en ce que** ladite chambre d'amplification est réalisée par au moins deux desdites strates superposées, dites strate supérieure (S2) et strate inférieure (S1), et **en ce que** ladite alcôve est réalisée dans l'une desdites deux strates uniquement.

4. Système selon la revendication 3, **caractérisé en ce que** ladite proéminence formant ladite alcôve (A1) est réalisée dans la strate inférieure.

5. Système selon la revendication 3 ou 4, **caractérisé en ce que** la chambre d'amplification (20) comporte une cavité principale réalisée dans la strate supérieure et une ou plusieurs cavités secondaires réalisées dans la strate inférieure et formant chacune une autre alcôve de ladite chambre.

6. Système selon l'une des revendications 1 à 5, **caractérisé en ce que** la deuxième zone présente au moins une partie opaque configurée pour ne pas laisser passer ledit signal optique.

7. Procédé d'analyse d'un échantillon biologique contenant des espèces biologiques, ledit procédé étant mis en œuvre par un système tel que défini dans l'une des revendications 1 à 6, **caractérisé en ce qu'**il consiste à :
- Injecter un échantillon fluidique dans la chambre d'amplification du dispositif micro-fluidique,
- Détecter grâce au capteur la présence d'un composé cible contenu dans ledit échantillon fluidique et situé dans la première zone de la chambre d'amplification,
- Détecter, avec un décalage temporel, la présence du composé de contrôle interne de réaction dans la première zone de la chambre d'amplification.

## Patentansprüche

1. Mikrofluidisches System zum Analysieren einer biologischen Probe, die biologische Spezies enthält, wobei das System umfasst:
- eine optische Detektionsvorrichtung, die eine Quelle umfasst, die dazu ausgestaltet ist, ein optisches Signal auszusenden, und mindestens einen Sensor, der eine Erfassungsfläche aufweist, die einen transparenten Lesebereich für das optische Signal definiert,
- eine mikrofluidische Vorrichtung, die beinhaltet:
∘ einen Träger, in dem eine sogenannte Amplifikationskammer (20) ausgeführt ist, in der eine Amplifikationsreaktion ausgeführt werden kann,
∘ einen Eintrittskanal, der in der Amplifikationskammer mündet,
- wobei die Amplifikationskammer mindestens einen ersten Bereich beinhaltet, der in dem Lesebereich des Sensors gelegen ist, und mindestens einen eine Nische (Ax) bildenden Vorsprung, in dem eine Verbindung platziert wird, die eine bekannte DNA-Sequenz oder einen Satz von DNA-Primern, die auf eine vorgegebene Ziel-DNA abzielen, beinhaltet, und ihre Amplifikation nach dem verwendeten Amplifikationsverfahren ermöglicht, **dadurch gekennzeichnet, dass** die Verbindung eine Verbindung zur internen Kontrolle der Amplifikationsreaktion ist und dass die Nische (Ax) dazu eingerichtet ist, außerhalb des Lesebereichs des Sensors gelegen zu sein, oder dazu ausgestaltet ist, für das optische Signal undurchlässig zu sein.

2. System nach Anspruch 1, **dadurch gekennzeichnet, dass** die Amplifikationskammer ein erstes Volumen beinhaltet, das einen ersten Querschnitt aufweist, und ein zweites Volumen, das einen zweiten Querschnitt aufweist, der in Bezug auf den ersten Querschnitt verengt ist, so dass ein Vorsprung gebildet wird, wobei der Vorsprung die Nische (Ax) bildet.

3. System nach Anspruch 1 oder 2, **dadurch gekennzeichnet, dass** der Träger mehrere übereinander liegende Schichten (S1, S2) beinhaltet und dass die Amplifikationskammer aus mindestens zwei der übereinander liegenden Schichten ausgeführt ist, obere Schicht (S2) und untere Schicht (S1) genannt, und dass die Nische nur in einer der beiden Schichten ausgeführt ist.

4. System nach Anspruch 3, **dadurch gekennzeichnet, dass** der die Nische (A1) bildende Vorsprung in der unteren Schicht ausgeführt ist.

5. System nach Anspruch 3 oder 4, **dadurch gekennzeichnet, dass** die Amplifikationskammer (20) eine Hauptkavität beinhaltet, die in der oberen Schicht ausgeführt ist, und eine oder mehrere Nebenkavitäten, die in der unteren Schicht ausgeführt sind und jeweils eine weitere Nische der Kammer bilden.

6. System nach einem der Ansprüche 1 bis 5, **dadurch gekennzeichnet, dass** der zweite Bereich mindestens einen undurchlässigen Teil aufweist, der dazu ausgestaltet ist, das optische Signal nicht durchzulassen.

7. Verfahren zum Analysieren einer biologischen Probe, die biologische Spezies enthält, wobei das Verfahren von einem System wie in einem der Ansprüche 1 bis 6 definiert durchgeführt wird, **dadurch gekennzeichnet, dass** es darin besteht:
- eine fluidische Probe in die Amplifikationskammer der mikrofluidischen Vorrichtung zu injizieren,
- mithilfe des Sensors das Vorhandensein einer Zielverbindung zu detektieren, die in der fluidischen Probe enthalten ist und in dem ersten Bereich der Amplifikationskammer gelegen ist,
- mit einem zeitlichen Versatz das Vorhandensein der Verbindung zur internen Kontrolle der Reaktion in dem ersten Bereich der Amplifikationskammer zu detektieren.

## Claims

1. Microfluidic system intended for the analysis of a biological sample containing biological species, said system comprising:
- an optical detection device comprising a source configured to emit an optical signal and at least one sensor having a capture surface defining a transparent optical signal reading zone,
- a microfluidic device which comprises:
∘ a support in which a chamber referred to as an amplification chamber (20), in which an amplification reaction can be carried out, is made,
∘ an input channel opening into said amplification chamber,
- the amplification chamber comprising at least one first zone located in the sensor reading zone and at least one protuberance forming a recess (Ax) in which is placed a compound which comprises a known DNA sequence or a set of DNA primers targeting a predefined DNA target, allowing the amplification thereof according to the amplification method used,
**characterized in that** the compound is a compound for internal control of the amplification reaction and **in that** said recess (Ax) is arranged to be located outside the sensor reading zone or is configured to be opaque to said optical signal.

2. System according to Claim 1, **characterized in that** the amplification chamber comprises a first volume having a first section and a second volume having a second section narrowed with respect to said first section so as to form a protuberance, said protuberance forming said recess (Ax).

3. System according to Claim 1 or 2, **characterized in that** said support comprises a plurality of superposed strata (S1, S2) and **in that** said amplification chamber is formed by at least two of said superposed strata, referred to as the upper stratum (S2) and the lower stratum (S1), and **in that** said recess is formed in only one of said two strata.

4. System according to Claim 3, **characterized in that** said protuberance forming said recess (A1) is made in the lower stratum.

5. System according to Claim 3 or 4, **characterized in that** the amplification chamber (20) comprises a main cavity made in the upper stratum and one or more secondary cavities made in the lower stratum and each forming another recess of said chamber.

6. System according to one of Claims 1 to 5, **characterized in that** the second zone has at least one opaque portion configured to not allow said optical signal to pass.

7. Method for analyzing a biological sample containing biological species, said method being implemented by a system as defined in one of Claims 1 to 6, **characterized in that** it consists in
- injecting a fluidic sample into the amplification chamber of the microfluidic device,
- detecting by means of the sensor the presence of a target compound contained in said fluidic sample and located in the first zone of the amplification chamber,
- detecting, with a time lag, the presence of the compound for internal control of the reaction, in the first zone of the amplification chamber.
